(19) Europäisches Patentamt
European Patent Office
Office européen des brevets

(11) Publication number: 0 143 655 B1

(12) # EUROPEAN PATENT SPECIFICATION

(45) Date of publication of patent specification: 26.10.88

(51) Int. Cl.$^4$: C 07 D 303/48, C 07 D 301/12

(21) Application number: 84308257.9

(22) Date of filing: 28.11.84

(54) **Process for the preparation of perfluoroalkene oxides.**

(30) Priority: 28.11.83 IT 2390883

(43) Date of publication of application:
05.06.85 Bulletin 85/23

(45) Publication of the grant of the patent:
26.10.88 Bulletin 88/43

(84) Designated Contracting States:
BE CH DE FR GB LI NL

(56) References cited:
BE-A- 751 149
US-A-3 358 003
US-A-3 992 432

(73) Proprietor: Montedison S.p.A.
31, Foro Buonaparte
I-20121 Milan (IT)

(72) Inventor: Bornengo, Giorgio
8 via Paletta
Novara (IT)
Inventor: Carlini, Filippo Maria
7 corso Torino
Novara (IT)
Inventor: Pontevivo, Michele
4, via Monte Nero
Novara (IT)
Inventor: Bottaccio, Giorgio
16 via Manin
Novara (IT)

(74) Representative: Whalley, Kevin et al
MARKS & CLERK 57/60 Lincoln's Inn Fields
London WC2A 3LS (GB)

EP 0 143 655 B1

Note: Within nine months from the publication of the mention of the grant of the European patent, any person may give notice to the European Patent Office of opposition to the European patent granted. Notice of opposition shall be filed in a written reasoned statement. It shall not be deemed to have been filed until the opposition fee has been paid. (Art. 99(1) European patent convention).

Courier Press, Leamington Spa, England.

**Description**

The present invention relates to a process for the preparation of perfluorinated alkene oxides, particularly perfluoropropene oxide, through the oxidization of perfluorinated alkenes, particularly perfluoropropene, with hydrogen peroxide.

From US—A—3358003 it is known to obtain perfluoropropene oxide by reacting perfluoropropene with an aqueous alkaline solution of hydrogen peroxide, possibly containing an organic water-miscible solvent.

However, by this process there are obtained only low yields of perfluoropropene oxide, of the order of 30%, and low degrees of selectivity, of the order of 35% under the most favourable conditions, with the formation of considerable quantities of fluorinated byproducts soluble in the aqueous phase and deriving from secondary reactions of the perfluoropropene and from subsequent decomposition in the aqueous alkaline medium of the thus formed perfluoropropene oxide.

When operating according to the above indicated process it is, moreover, necessary to maintain conversion values not exceeding 90% because the selectivity in perfluoropropene oxide decreases at conversions between 90% and 100%.

It is noted that the use of phase transfer catalysts is known in processes for the oxidation of olefines to their oxides, see US—A—3992432. Also, the introduction of such a catalyst into the reaction is known from US—A—3358003 and BE—A—751149.

Thus, the present invention aims to obtain, particularly in the oxidization of perfluoropropene oxide with hydrogen peroxide, high conversion values of the perfluoropropene greater than 90% and high selectivities in perfluoropropene oxide and consequently high yields in perfluoropropene oxide greater than 60%, while minimising the quantity of fluorinated byproducts derived from secondary reactions of the perfluoropropene or from the decomposition of the perfluoropropene oxide in the aqueous alkaline medium.

It has now been found that satisfactory results may be achieved when the oxidation reaction of perfluorinated alkenes, particularly perfluoropropene, with alkaline hydrogen peroxide is made to occur in the presence of a phase-transfer catalyst and if the alkalinization of the reaction mixture is carried out gradually so as to maintain the reaction temperature constant at a pre-established value from −10° to −60°C.

Thus, the present invention provides a process for the preparation of a perfluorinated alkene oxide, particularly perfluoropropene oxide, by the reaction of a perfluorinated alkene, particularly perfluoropropene, with hydrogen peroxide in an aqueous alkaline medium in the presence of a water-miscible organic solvent, characterized in that a mixture of said perfluorinated alkene, an aqueous solution of hydrogen peroxide, an organic solvent miscible with water, in an inert organic solvent substantially immiscible with water, and a phase-transfer catalyst selected from quaternary ammonium salts, quaternary phosphonium salts and lipophilic complexing agents for cations, wherein the perfluorinated alkene, the perfluorinated alkene oxide, and the said phase-transfer catalyst are at least partially soluble in the said substantially water-immiscible solvent, said mixture being maintained at a temperature from −10°C to −60°C, under stirring, is gradually additioned with an aqueous solution of an inorganic base, during a time period from 15 minutes to 6 hours, selected so as to maintain the said temperature constant.

Reference will in particularly be made in the following description to the preparation of perfluoropropene oxide from perfluoropropene, but as mentioned subsequently the process of the present invention may be usefully extended to the preparation of oxides of other perfluorinated alkenes.

The volume ratio of the organic phase, comprising the perfluoropropene, the catalyst and the water-immiscible organic solvent, to the aqueous phase comprising the aqueous solution of hydrogen peroxide, the water-miscible organic solvent and the basic aqueous solution, present in the reaction mixture, is not critical and is preferably from 0.01 to 5.

The hydrogen peroxide is preferably present in the reaction mixture in an amount from 1 to 10 mols per mol of perfluoropropene, more preferably in an amount from 3 to 5 mols per mol of perfluoropropene, normally in the form of a 35% aqueous solution.

The inorganic base may be suitably selected from the hydroxides and the basic salts of alkali and alkaline-earth metals and of ammonium, such as for example sodium hydroxide, potassium hydroxide, ammonium hydroxide, sodium bicarbonate and sodium acetate.

The inorganic base is admixed with the reaction system in the form of an aqueous solution, preferably in an amount from 0.01 to 10 mols per mol of perfluoropropene.

By suitably adjusting the inorganic base/perfluoropropene molar ratio, it is possible to obtain the desired degree of conversion, for instance with a molar ratio of 1—1.2 there is achieved a conversion of about 90%, while with a ratio of 2—2.5 there is attained a conversion nearing 100%.

As water-miscible organic solvents there may be suitably used aliphatic alcohols, ketones, aldehydes and esters, in particular methanol, ethanol, acetone and acetaldehyde.

The quantity to be employed is not critical and depends on the reaction conditions.

Their use allows the reaction to be carried out at low temperatures, for example from −30° to −50°C.

The substantially water-immiscible organic solvent preferably has a freezing point below −60°C or at least lower than the reaction temperature, while in it the perfluoropropene, the perfluoropropene oxide and

the catalyst must be sufficiently soluble.

The presence of such solvent will minimise the secondary reactions of the perfluoropropene oxide in the aqueous alkaline phase and consequently permit an increase in yield to be obtained.

Such solvent is preferably used in an amount from 0.1 to 20 mols per mol of perfluoropropene, and is chosen suitably as a function of its immiscibility in the aqueous phase and of the solubility of the catalyst, the perfluoropropene and the perfluoropropene oxide in the solvent.

Suitable such solvents are, for example, ethers such as diisopropyl and di-n-butyl ether; halogenated compounds such as carbon tetrachloride, 1,2-dichloroethane, methylene chloride, and chloroform; chlorofluorocarbons such as 1,2-dichloro-1,1,2,2-tetrafluoroethane, 1,1,2-trichloro-1,2,2,2-trifluoroethane, and 1,1,2,2-tetrachloro-1,2-difluoroethane; and fluorocarbons such as perfluorocyclobutane, perfluorodimethylcyclobutane, perfluorohexane and hexafluorobenzene.

Particularly preferred are the solvents in which both the perfluoropropene as well as the perfluoropropene oxide have a high solubility, such as chloro-fluorocarbons (fluorinated-chlorinated hydrocarbons), as well as the solvents in which the catalyst has a high solubility, such as chlorinated compounds.

The phase-transfer catalyst, selected from quaternary ammonium salts, quaternary phosphonium salts and lipophilic complexing agents for cations, preferably has a good solubility in the organic phase and is preferably used in an amount from 0.001 mols to 10 mols per mol of perfluoropropene.

The quaternary ammonium or phosphonium salts that may be used according to the present invention, are represented by the following general formulae:

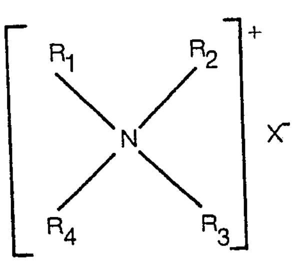 (I) 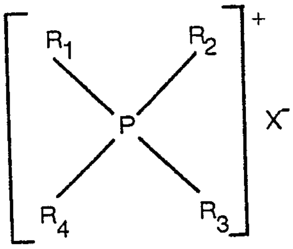 (II)

wherein: $R_1$, $R_2$, $R_3$ and $R_4$ may be the same as or different from each other and may each represent a hydrocarbon group which may or may not be substituted by a functional group inert under reaction conditions. The type and the length of the hydrocarbon group are suitably chosen having regard to their solubility in the organic phase, to the composition of the reaction mixture and to the desired reaction rate.

Examples of such hydrocarbon groups include alkyl, alkenyl, cycloalkyl, cycloalkenyl, aryl, aralkyl, and alkenaryl groups. Amongst these are particularly preferred the alkyl, aryl and aryl-alkyl groups.

As far as the length of the hydrocarbon chains is concerned, the total number of carbon atoms contained in $R_1$, $R_2$, $R_3$ and $R_4$ is preferably from 6 to 100, more preferably 10 to 50.

In the hydrocarbon chain there may be present inert functional groups such as halogens, acyl, carboxy, and ester groups.

$R_1$, $R_2$, $R_3$ and $R_4$ may combine with each other to form a nitrogen containing heterocyclic ring or they may form part of a polymeric compound.

Quaternary ammonium ions that may be conveniently used include: tetraethylammonium, tetra-n-propylammonium, tetra-n-butyl-ammonium, tri-n-octyl-methylammonium, cetyl-trimethylammonium, benzyl-trimethylammonium, benzyl-triethylammonium, cetyl-benzyl-dimethylammonium, cetylpyridinium, n-dodecyl-pyridinium, phenyl-trimethyl-ammonium, phenyl-triethylammonium, N-benzyl-picolinium, and 2-6-di-ter-butyl pyridinium.

Amongst these ions the preferred ones are those having alkyl groups with a long chain and pyridinium ions.

Phosphonium ions that may be conveniently used include: tetraethylphosphonium, tetra-n-butylphosphonium, tri-n-octylethylphosphonium, cetyl-triethylphosphonium, cetyl-tri-n-butylphosphonium, n-butyl-triphenyl-phosphonium, n-amyl-triphenylphosphonium, n-hexyl-triphenylphosphonium, n-heptyl-triphenylphosphonium, methyl-triphenylphosphonium, benzyl-triphenylphosphonium, tetraphenyl phosphonium and acetonyl-triphenyl phosphonium.

Amongst these phosphonium ions, there are preferred those having alkyl groups with a long chain as well as those carrying three phenyl groups.

The $X^-$ ions, in formulae (I) and (II), are not subject to particular limitations and there may be conveniently used halogen ions of other mineral acids, ions of organic acids, and hydroxy ions.

Examples of such ions are: chloride, bromide, iodide, fluoride, hydrogen sulphate, sulphate, nitrate, phosphate, perchlorate, hydroxy, acetate, benzoate, benzenesulphonate and p-toluene-sulphonate. Amongst these the chloride and the hydroxy ions are the preferred ones.

The lipophilic complexing agents for cations are selected from compounds which, besides having a certain solubility in the organic phase, are capable of forming stable complexes with the cations present in the reaction mixture.

Typical examples of such compounds, which may be used in the present invention, are represented by

the oxygenated ring-shaped compounds, commonly called "crown ethers", and by polyethyleneglycol or derivatives therefrom.

The "crown ethers", described, for instance, in Pedersen, in the Jr. of Amer. Chem. Soc., *89,* 2495, 7017 (1967), have the capacity of stably coordinating alkali and alkaline-earth metal ions, and may almost totally be represented, also including their substituted derivatives, by the general formula:

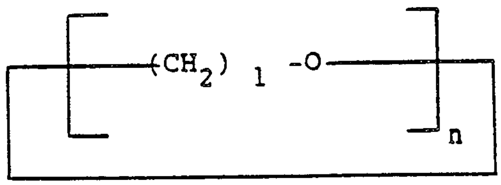

(III)

wherein: n is an integer from 4 to 20 and where 1, in each of the n-groups $(CH_2)_1$, may assume equal or different values selected from 2, 3 and 4.

Examples of some of the crown ethers (in accordance with the Pedersen nomenclature) which may be used according to this invention, are: 18-crown-6, dicyclohexyl-18-crown-6, dibenzo-18-crown-6, benzo-15-crown-5, dibenzo-15-crown-5, dibenzo-21-crown-7, dibenzo-24-crown-8, dibenzo-30-crown-10, and dicyclohexyl-24-crown-8. The polyethyleneglycols, that may be used according to the present invention, may have various degrees of polymerization.

Polyethyleneglycol derivatives, that may be used according to the present invention, include the compounds represented by formulae (IV) and (V), their substituted derivatives, the copolymers of ethylene oxide with other monomers, and in general the compounds containing the structure of the polyethyleneglycol.

$$R_4-O-(CH_2-CH_2)_t-H \qquad (IV)$$

$$R_5-O-(CH_2-CH_2-O)_u-R_6 \qquad (V)$$

wherein: t is an integer greater than or equal to 5, while u is an integer greater than or equal to 3, and $R_4$, $R_5$ and $R_6$ represent substituted or unsubstituted hydrocarbon groups containing from 1 to 80 carbon atoms.

Amongst the polyethyleneglycols there are preferred: alkyl-phenyl-polyethyleneglycol (TRITON X 100®); polyethylene-lauryl-ether (BRIJ 35®) and derivatives of the formula:

$$n-C_{16}H_{33}-O-(CH_2-CH_2-O)_{12}H,$$

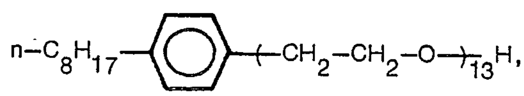

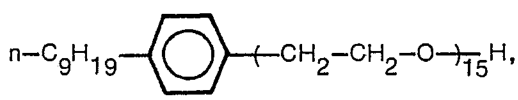

$$CH_3O-(CH_2-CH_2-O)_3CH_3$$

$$n-C_4H_9O-(CH_2=CH_2-O)_{10}n-C_4H_9$$

The reaction temperature is not critical and may be selected within a range from −10° to −60°C, depending on the composition of the reaction mixture and on the desired reaction rate.

At the end of the reaction, the organic phase is separated from the aqueous phase, and the perfluoropropene oxide is isolated from the organic phase by means of simple separation techniques, for example by distillation.

The process of the present invention, contrary to the process described in U.S. Patent No. 3,358,003, allows perfluoropropene oxide to be obtained at high selectivities even at high conversions of the perfluoropropene, with a good control of the reaction, while avoiding the decomposition of the perfluoropropene oxide that is formed as well as minimizing the formation of byproducts.

It is thus possible, operating at high conversions, to avoid long and costly separation steps of the perfluoropropene oxide from the unreacted perfluoropropene, as well as avoiding the perfluoropropene recycling stage, with operational and economical advantages. Thus, the present process allows perfluoropropene oxide to be prepared in high yields and by means of a simplified and inexpensive procedure suitable for being applied on a commercial scale.

The invention will be further described with reference to the following illustrative Examples.

Example 1

Into a 2.5 lt reactor, fitted with a thermostatically controlled cooling sleeve with the forced circulation of a refrigerating mixture ($CH_3$—CO—$CH_3$ + solid $CO_2$) and provided with a stirrer, there were loaded 297 g of methanol, 289 g of a 36% hydrogen peroxide solution and 1.8 g (4.5 mmols) of tetrabutyl-ammonium hydroxide (as a phase transfer catalyst) preliminarily dissolved in 132 g of methylene chloride.

The reactor was then cooled down to −50°C and into it were then introduced 100 g of perfluoropropene.

Thereupon, by means of a dropping funnel and under vigorous stirring, there was added, during a time period of about 90 min., into the mixture of solution of 40 g of KOH in 80 ml of $H_2O$. The reaction was then completed maintaining the mixture under constant stirring for 3 hours at −50°C.

Then, by raising the temperature slowly from −50°C to +20°C, the gas was removed from the reactor and separated from the organic solvent by means of a condenser kept at −20°C. The perfluoropropene oxide was then gathered in a trap cooled down to −70°C.

Chromatographic gas analysis showed that the final gaseous mixture contained 86% of perfluoropropene oxide, with a conversion of perfluoropropene of 95%, a selectivity of 74% and a yield in perfluoropropene oxide of 70%.

Examples 2—12

The same procedure as that followed in example 1 was repeated, except that instead of tetrabutylammonium hydroxide, there were used 4.5 mmols of the phase-transfer catalysts given in Table 1.

The conversions and yields obtained are given in Table 1.

TABLE 1

| Example No. | Catalyst | Conversion of Perfluoropropene | Selectivity in perfluoro propene oxide |
|---|---|---|---|
| 2 | $(C_8H_{17})_3CH_3N$ Cl | 93 | 66 |
| 3 | $(C_4H_9)_4N$ Cl | 91 | 62 |
| 4 | $(C_3H_7)_4N$ OH | 80 | 70 |
| 5 | $(C_2H_5)_4N$ Cl | 92 | 52 |
| 6 | $(C_2H_5)_4NI$ | 94 | 50 |
| 7 | $(CH_3)_3(C_6H_5CH_2)N$ OH | 92 | 56 |
| 8 | $(CH_3)_3(C_6H_5CH_2)N$ Cl | 95 | 48 |
| 9 | $(C_6H_5)_3(CH_3$—CO—$CH_2)P$ Cl | 90 | 55 |
| 10 | $(C_4H_9)_4P$ Cl | 93 | 57 |
| 11 | dibenzo-18-crown-6 | 88 | 70 |
| 12 | 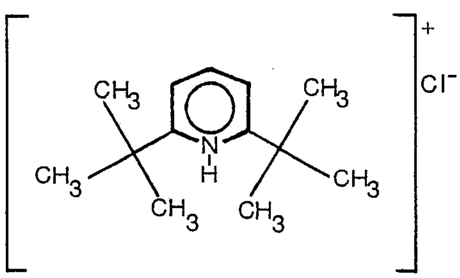 | 99 | 68 |

Example 13

The same procedure as described in example 1 was repeated, but using as a phase-transfer catalyst 7 grams of polyoxyethylenelaurylether (BRIJ 35®) instead of tetrabutylammonium hydroxide. Thereby there was obtained a conversion of the perfluoropropene of 80% and a selectivity in perfluoropropene oxide of 65%.

Example 14

Again there was repeated the procedure of example 1, but using 7 grams of alkylphenylpolyethyleneglycol (TRITON X 100®) as a phase-transfer catalyst. Thereby there was obtained a conversion of the perfluoropropene of 82% and a selectivity in perfluoropropene oxide of 66%.

Example 15

There was repeated the procedure of example 2, but using 156 grams of 1,1,2-trichloro-1,2,2-trifluoroethane (FREON® 113) instead of 132 grams of methylene chloride. Thereby there was obtained a conversion of the perfluoropropene of 84% and a selectivity in perfluoropropene oxide of 66%.

Example 16

The procedure of example 12 was repeated, but using 156 grams of 1,1,2-trichloro-1,2,2-trifluoroethane instead of 132 grams of methylene chloride. Thereby there was obtained a conversion of the perfluoropropene of 98% and a selectivity in perfluoropropene oxide of 65%.

Example 17 (comparison)

The procedure of example 1 was repeated, except that neither the phase-transfer catalyst not the organic solvent immiscible with the aqueous phase, represented by the methylene chloride, were used.

Gas-chromatographic analysis showed that the gaseous phase contained 77% of perfluoropropene oxide, with a conversion of perfluoropropene of 86% and a selectivity in perfluoropropene oxide of 44%, while the yield was 38%.

**Claims**

1. A process for the preparation of a perfluorinated alkene oxide, particularly perfluoropropene oxide, by the reaction of a perfluorinated alkene, particularly perfluoropropene, with hydrogen peroxide in an aqueous alkaline medium in the presence of a water-miscible organic solvent, characterized in that a mixture of said perfluorinated alkene, an aqueous solution of hydrogen peroxide, an organic solvent miscible with water, an inert organic solvent substantially immiscible with water, and a phase-transfer catalyst selected from quaternary ammonium salts, quaternary phosphonium salts and lipophilic complexing agents for cations, wherein the perfluorinated alkene, the perfluorinated alkene oxide, and the said phase-transfer catalyst are at least partially soluble in the said substantially water-immiscible solvent, said mixture being maintained at a temperature from −10°C to −60°C, under stirring, is gradually additioned with an aqueous solution of an inorganic base, during a time period from 15 minutes to 6 hours, selected so as to maintain the said temperature constant.

2. A process as claimed in claim 1, characterized in that the hydrogen peroxide is contained in the mixture in an amount from 1 to 10 mols per mol of the perfluorinated alkene, particularly perfluoropropene.

3. A process as claimed in claim 2, characterized in that the hydrogen peroxide is contained in the mixture in an amount from 3 to 5 mols per mol of the perfluorinated alkene, particularly perfluoropropene.

4. A process as claimed in any of claims 1 to 3, characterized in that the inorganic base is additioned in an amount from 0.01 to 10 mols per mol of the perfluorinated alkene, particularly perfluoropropene.

5. A process as claimed in any of claims 1 to 4, characterized in that the organic water-miscible solvent is selected from methanol, ethanol, acetone and acetaldehyde.

6. A process as claimed in any of claims 1 to 5, characterized in that the organic solvent substantially immiscible with water is contained in the mixture in an amount from 0.1 to 20 mols of the perfluorinated alkene, particularly perfluoropropene.

7. A process as claimed in any of claims 1 to 6, characterized in that the organic solvent substantially immiscible with water is selected from chlorinated compounds and chloro-fluorocarbons.

8. A process as claimed in any of claims 1 to 7, characterized in that the phase-transfer catalyst is contained in the mixture in an amount from 0.001 to 10 mols per mol of the perfluorinated alkene, particularly perfluoropropene.

9. A process as claimed in any of claims 1 to 8, characterized in that the quaternary ammonium and phosphonium salts contain from 6 to 100 carbon atoms.

10. A process as claimed in any of claims 1 to 8, characterized in that the lipophilic complexing agent is selected from oxygenated ringed compounds of the formula:

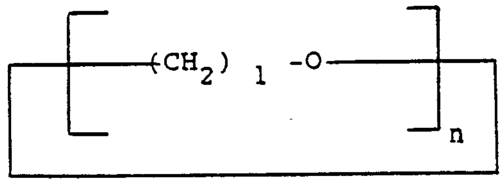

(III)

wherein: n is a integer from 4 to 20 and where 1, in each of the n-groups $(CH_2)_1$, may assume values equal to or different from each other selected from 2, 3 and 4.

11. A process as claimed in any of claims 1 to 8, characterized in that the lipophilic complexing agent is polyethyleneglycol or a derivative thereof.

12. A process as claimed in any of claims 1 to 11, characterized in that the ratio by volume between the organic phase and the aqueous phase present in the reaction mixture is from 0.01 to 5.

**Patentansprüche**

1. Verfahren zur Herstellung eines perfluorierten Alkenoxids, insbesondere Perfluorpropenoxid, durch Umsetzung eines perfluorierten Alkens, insbesondere Perfluoropropen, mit Wasserstoffperoxid in einem wässrigen alkalischen Medium in Anwesenheit eines mit Wasser mischbaren organischen Lösungsmittels, dadurch gekennzeichnet, daß eine Mischung aus dem gennannten perfluorierten Alken, einer wässrigen Lösung von Wasserstoffperoxid, einem organischen, mit Wasser mischbaren Lösungsmittel, einem inerten organischen, mit Wasser praktisch nicht mischbaren Lösungsmittel und einem Phasenübertragungs-Katalysator, ausgewählt aus quaternären Ammoniumsalzen, quaternären Phosphoniumsalzen und lipophilen, Komplex bildenden Mitteln für Kationen, wobei das perfluorierte Alken, das perfluorierte Alkenoxid und der Phasenübertragungs-Katalysator mindestens teilweise im genannten, mit Wasser praktisch nicht mischbaren Lösungsmittel löslich sind und die Mischung, die auf einer Temperatur von −10°C bis −60°C unter Rühren gehalten wird, mit einer wässrigen Lösung einer anorganischen Base während einer Dauer von 15 min bis 6 h, die so ausgewählt ist, daß diese Temperatur konstant gehalten wird, allmählich versetzt wird.

2. Verfahren nach Anspruch 1, dadurch gekennzeichnet, daß das Wasserstoffperoxid in der Mischung in einer Menge von 1 bis 10 Mol pro Mol des perfluorierten Alkens, insbesondere Perfluorpropen, enthalten ist.

3. Verfahren nach Anspruch 2, dadurch gekennzeichnet, daß das Wasserstoffperoxid in der Mischung in einer Menge von 3 bis 5 Mol pro Mol des perfluorierten Alkens, insbesondere Perfluorpropen, enthalten ist.

4. Verfahren nach irgendeinem der Ansprüche 1 bis 3, dadurch gekennzeichnet, daß die anorganische Base in einer Menge von 0,11 bis 10 Mol pro Mol des perfluorierten Alkens, insbesondere Perfluorpropen, zugefügt wird.

5. Verfahren nach irgendeinem der Ansprüche 1 bis 4, dadurch gekennzeichnet, daß das organische, mit Wasser mischbare Lösungsmittel ausgewählt wird aus Methanol, Ethanol, Aceton und Acetaldehyd.

6. Verfahren nach irgendeinem der Ansprüche 1 bis 5, dadurch gekennzeichnet, daß das organische, mit Wasser praktisch nicht mischbare Lösungsmittel in der Mischung in einer Menge von 0,1 bis 20 Mol des perfluorierten Alkens, insbesondere Perfluoropropen, enthalten ist.

7. Verfahren nach irgendeinem der Ansprüche 1 bis 6, dadurch gekennzeichnet, daß das organische, mit Wasser praktisch nicht mischbare Lösungsmittel ausgerwählt wird aus chlorierten Verbindungen und Chlorfluorkohlenstoffen.

8. Verfahren nach irgendeinem der Ansprüche 1 bis 7, dadurch gekennzeichnet, daß der Phasenübertragungs-Katalysator in der Mischung in einer Menge von 0,001 bis 10 Mol pro Mol des perfluorierten Alkens, insbesondere Perfluorpropen, enthalten ist.

9. Verfahren nach irgendeinem der Ansprüche 1 bis 8, dadurch gekennzeichnet, daß die quaternären Ammonium- und Phosphoniumsalze von 6 bis 100 Kohlenstoffatome enthalten.

10. Verfahren nach irgendeinem der Ansprüche 1 bis 8, dadurch gekennzeichnet, daß das lipophile, Komplex bildende Mittel ausgewählt wird aus sauerstoffhaltigen, zum Ring geschlossenen Verbindungen der Formel

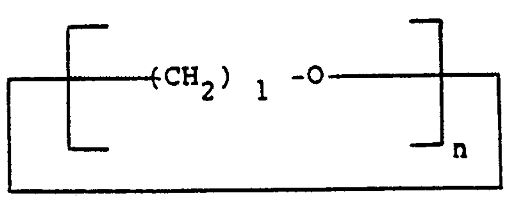

(III)

worin n eine ganze Zahl von 4 bis 20 ist und 1 in jeder der n Gruppen $(CH_2)_1$ Werte annehmen kann, die

gleich oder voneinander verschieden sind und 2, 3 bzw. 4 betgragen.

11. Verfahren nach irgendeinem der Ansprüche 1 bis 8, dadurch gekennzeichnet, daß das lipophile, Komplex bildende Mittel Polyethylenglykol oder ein Derivat desselben ist.

12. Verfahren nach irgendeinem der Ansprüche 1 bis 11, dadurch gekennzeichnet, daß das Volumenverhältnis zwischen der organischen Phase und der in der Reaktionsmischung anwesenden, wässrigen Phase von 0,01 bis 5 beträgt.

**Revendications**

1. Un procédé de préparation d'oxyde de perfluoroalkène, notamment d'oxyde de perfluoropropène, par réaction d'un perfluoroalkène, notamment de perfluoropropène, avec du peroxyde d'hydrogène dans un milieu alcalin aqueux en présence d'un solvant organique miscible à l'eau, caractérisé en ce qu'un mélange de ce perfluoroalkène, d'une solution aqueuse de peroxyde d'hydrogène, d'un solvant organique miscible à l'eau, d'un solvant organique inerte sensiblement non miscible à l'eau, et d'un catalyseur de transfert de phase choisi parmi les sels d'ammonium quaternaires, les sels de phosphonium quaternaires et des agents complexants lipophiles des cations, mélange dans lequel le perfluoroalkène, l'oxyde de perfluoroalkène et ce catalyseur de transfert de phase sont au moins partiellement solubles dans ce solvant sensiblement non miscible à l'eau, est maintenu à une température comprise entre −10°C et −60°C, sous agitation, et est progressivement additionné d'une solution aqueuse d'une base minérale, pendant une période de temps comprise entre 15 minutes et 6 heurs, de sorte que cette température soit maintenue constante.

2. Un procédé selon la revendication 1, caractérisé en ce que le peroxyde d'hydrogène est contenu dans le mélange en une quantité comprise entre 1 et 10 moles/mole de perfluoroalkène, notamment de perfluoropropène.

3. Un procédé selon la revendication 2, caractérisé en ce que le peroxyde d'hydrogène est contenu dans le mélange en une quantité comprise entre 3 et 5 moles/mole de perfluoroalkène, notamment de perfluoropropène.

4. Un procédé selon l'une quelconque des revendications 1 à 3, caractérisé en ce que la base minérale est additionnée en une quantité comprise entre 0,01 et 10 moles/mole de perfluoroalkène, notamment de perfluoropropène.

5. Un procédé selon l'une quelconque des revendications 1 à 5, caractérisé en ce que le solvant organique miscible à l'eau est choisi parmi: méthanol, éthanol, acétone et acétaldéhyde.

6. Un procédé selon l'une quelconque des revendications 1 à 5, caractérisé en ce que le solvant organique sensiblement non miscible à l'eau est contenu dans le mélange en une quantité comprise entre 0,1 et 20 moles de perfluoroalkène, notamment de perfluoropropène.

7. Un procédé selon l'une quelconque des revendications 1 à 6, caractérisé en ce que le solvant organique sensiblemenet non miscible à l'eau est choisi parmi les dérivés chlorés et les chlorofluorocarbones.

8. Un procédé selon l'une quelconque des revendications 1 à 7, caractérisé en ce que le catalyseur de transfert de phase est contenu dans le mélange en une quantité comprise entre 0,001 et 10 moles/mole de perfluoroalkène, notamment de perfluoropropène.

9. Un procédé selon l'une quelconque des revendications 1 à 8, caractérisé en ce que les sels d'ammonium et de phosphonium quaternaires renferment de 6 à 100 atomes de carbone.

10. Un procédé selon l'une quelconque des revendications 1 à 8, caractérisé en ce que l'agent complexant lipophile est choisi parmi les dérives cycliques oxygénés de formule:

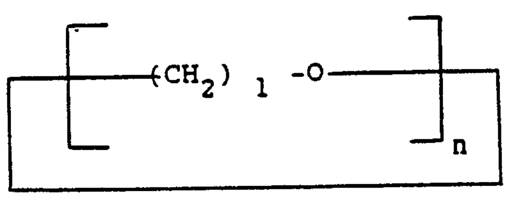

(III)

dans laquelle:

n représente un nombre entier compris entre 4 et 20; et

1 dans chacun des n-groupes $(CH_2)_1$, peut prendre des valeurs identiques ou différentes l'une de l'autre égales à 2, 3 ou 4.

11. Un procédé selon l'une quelconque des revendications 1 à 8, caractérisé en ce que l'agent complexant lipophile est le polyéthylèneglycol ou un de ses dérivés.

12. Un procédé selon l'une quelconque des revendications 1 à 11, caractérisé en ce que le rapport en volume entre la phase organique et la phase aqueuse présente dans le mélange réactionnel est compris entre 0,01 et 5.